(19) Europäisches Patentamt
European Patent Office
Office européen des brevets

(11) Veröffentlichungsnummer: **0 147 852**
**A2**

(12) # EUROPÄISCHE PATENTANMELDUNG

(21) Anmeldenummer: 84116374.4

(22) Anmeldetag: 27.12.84

(51) Int. Cl.[4]: C 07 D 493/22
A 01 N 43/00, A 61 K 31/355
//(C07D493/22, 313:08, 311:00, 311:00, 307:00)

(30) Priorität: 30.12.83 CH 6987/83
28.08.84 CH 4118/84

(43) Veröffentlichungstag der Anmeldung: 10.07.85 Patentblatt 85/28

(84) Benannte Vertragsstaaten: AT BE CH DE FR GB IT LI NL SE

(71) Anmelder: CIBA-GEIGY AG
Postfach
CH-4002 Basel(CH)

(72) Erfinder: Frei, Bruno, Dr.
Breitibachstrasse 10
CH-8600 Dübendorf(CH)

(72) Erfinder: Mereyala, Hari Babu, Dr.
Kriesbachstrasse 10
CH-8600 Dübendorf(CH)

(74) Vertreter: Zumstein, Fritz, Dr. et al,
Bräuhausstrasse 4
D-8000 München 2(DE)

(54) **Ektoparasitizides und insektizides Mittel.**

(57) Neue 14-Azido-15-hydroxi-Milbemycin-Derivate und 15-Hydroxi-$\Delta^{13,14}$-Milbemycin-Derivate lassen sich mit dem Komplex-Reagenz

$[HN_3]_m/[Al(Ethyl)_3]_n$,

worin m under n unabhängig voneinander die Zahl 1 oder 2 oder einen Zahlenwert zwischen 1 und 2 darstellen, aus den entsprechenden 14,15-Epoxi-Milbemycin-Derivaten in wasserfreier Phase und bei −30°C bis +10°C herstellen. Sie dienen als Wirkstoffe zur Bekämpfung von Arthropodenarten, wie Schadinsekten oder tierische Ekto- oder Endoparasiten und werden in Form von Mitteln angewandt. Die Verbindungen dienen auch als Zwischenprodukte zur weiteren Derivatisierung von Milbemycin-Derivaten.



Croydon Printing Company Ltd.

CIBA-GEIGY AG
Basel (Schweiz)

5-14721/1+2 /=

## Ektoparasitizides und insektizides Mittel

Die vorliegende Erfindung betrifft Milbemycin-Derivate der Formeln $1^*$ und $2^*$, ihre Herstellung und ihre Verwendung zur weiteren Derivatisierung von Milbemycinen sowie ihre Verwendung zur Bekämpfung von Schädlingen wie Ekto- und Endoparasiten, ferner Schädlingsbekämpfungsmittel, die mindestens eine dieser Verbindungen als Wirkstoff enthalten.

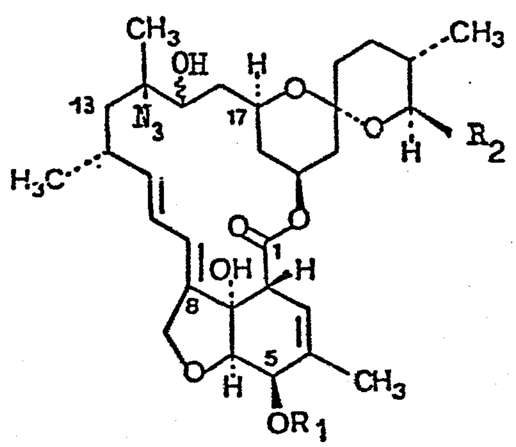

$1^*$

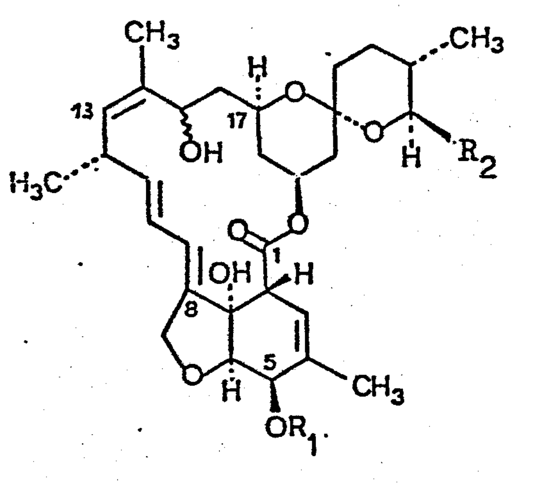

$2^*$

In den Formeln $1^*$ und $2^*$ bedeuten

$R_1$ Wasserstoff, eine Silyl- oder Acylgruppe, und

$R_2$ Methyl, Ethyl, Isopropyl oder sek.Butyl.

Verbindungen, worin $R_2$ sek.Butyl darstellt, sollen hier und im folgenden gleichfalls zu den Milbemycin-Derivaten gerechnet werden, obwohl sie nach der üblichen Systematik nicht darunter fallen , sondern von Avermectin-Derivaten abgeleitet sind. Avermectin-Aglykone (mit einer α-ständigen OH-Gruppe in 13-Position) lassen sich jedoch gemäss US-PS

4.173.571 in Milbemycin-Derivate überführen.

Im Rahmen vorliegender Erfindung sind Verbindungen der Formel 2* bevorzugt.

Als Acylgruppe für $R_1$ kommen $R_3$-CO-Reste und $R_4$-$SO_2$-Reste in Frage, wobei $R_3$ vorzugsweise einen unsubstituierten oder halogenierten $C_1$-$C_6$-aliphatischen Rest oder unsubstituiertes oder durch $C_1$-$C_4$-Alkyl oder Halogen substituiertes Phenyl bedeutet und $R_4$ einen $C_1$-$C_4$-Alkylrest oder einen unsubstituierten oder durch Methyl, Chlor oder Nitro substituierten Phenylrest darstellt.

Bevorzugt sind Verbindungen der Formeln 1* und 2*, worin $R_1$ Wasserstoff, einen $R_3$-CO-Rest oder $R_4$-$SO_2$-Rest bedeutet, in welchen $R_3$ einen $C_1$-$C_4$-Alkylrest oder eine unsubstituierte oder durch Methyl oder Chlor substituierte Phenylgruppe darstellt und $R_4$ Methyl, Ethyl, Phenyl, p-Tolyl, o-Nitrophenyl, p-Chlorphenyl ist, während $R_2$ Methyl, Ethyl, Isopropyl oder sek.Butyl bedeutet.

Als Beispiele für $R_3$, die keine Limitierungen darstellen sollen, seien genannt Methyl, Ethyl, Propyl, Isopropyl, tert.Butyl, Phenyl, p-Chlorphenyl, p-Tolyl.

Als Silyl-Gruppen kommen solche der Formel

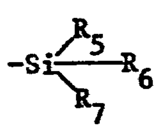

in Frage, worin $R_5$ einen $C_1$-$C_4$-aliphatischen Rest oder Benzyl und $R_6$ und $R_7$ unabhängig voneinander einen $C_1$-$C_4$-aliphatischen Rest, Benzyl oder Phenyl bedeuten.

Eine andere Gruppe bevorzugter Verbindungen der Formel 1* und 2* sind solche, worin $R_1$ Wasserstoff oder die vorgenannte Silylgruppe

darstellt, in der $R_5$ Methyl, Ethyl, Propyl, Isopropyl, tert.Butyl, und $R_6$ und $R_7$ unabhängig Methyl, Ethyl, Isopropyl, tert.Butyl, Phenyl oder Benzyl bedeuten, während $R_2$ Methyl, Ethyl, Isopropyl oder sek.Butyl ist.

Beispiele für Silyl-Gruppen sind Trimethylsilyl, tris(tert.-Butyl)silyl, Diphenyl-tert.butylsilyl, bis(Isopropyl)methylsilyl und besonders tert.Butyl-dimethylsilyl.

Verbindungen der Formel $2^*$, worin $R_1$ Wasserstoff und $R_2$ Methyl, Ethyl, Isopropyl oder sek.Butyl bedeuten, sind besonders bevorzugt.

Durch übliche Acylierung der 5-OH-Gruppe mit den entsprechenden Acylhalogeniden oder Acylanhydriden oder durch Reaktion der 5-OH-Gruppe mit dem entsprechend substituierten Silan-Derivat der Formel

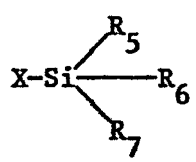

werden jene vorgenannten Derivate und solche der untengenannten Formel $3^*$ hergestellt, bei denen $R_1$ eine andere Bedeutung als Wasserstoff hat, und worin $R_5$, $R_6$ und $R_7$ die für die Formeln $1^*$ und $2^*$ genannten Reste darstellen, wobei der Begriff Acylhalogenid für Acylchlorid oder Acylbromid steht und wobei X eine Silylabgangsgruppe bedeutet. Zu den Silylabgangsgruppen X zählen beispielsweise Bromid, Chlorid, Cyanid, Azid, Acetamid, Trifluoracetat, Trifluormethansulfonat. Diese Aufzählung stellt keine Limitierung dar, der Fachmann kennt weitere typische Silylabgangsgruppen.

5-O-Acylierungen und 5-O-Silylierungen werden in wasserfreiem Milieu, vorzugsweise in inerten Lösungsmitteln und besonders bevorzugt in

aprotischen Lösungsmitteln durchgeführt. Die Reaktion läuft vorteilhaft im Temperaturbereich von 0°C bis 80°C, bevorzugt bei 10°C bis 40°C, ab. Vorzugsweise wird eine organische Base zugegeben. Es kommen als solche beispielsweise tertiäre Amine wie Triethylamin, Triethylendiamin, Triazol und bevorzugt Pyridin, Imidazol oder 1,8-Diazabicyclo[5.4.o]-undec-7-en (DBU) in Frage.

Die Entfernung dieser Silyl- und Acylreste $R_1$ in der 5-Position geschieht durch selektive milde Hydrolyse (⟶ R=H), die dem Fachmann geläufig ist, z.B. durch Verseifen mit einer verdünnten Arylsulfonsäure.

Die Verbindungen der Formeln $1^*$ und $2^*$ lassen sich nebeneinander gezielt aus 14,15-Epoxi-Milbemycinen der Formel $3^*$ gewinnen, worin $R_1$ und $R_2$ die für die Formeln $1^*$ und $2^*$ genannten Bedeutungen haben,

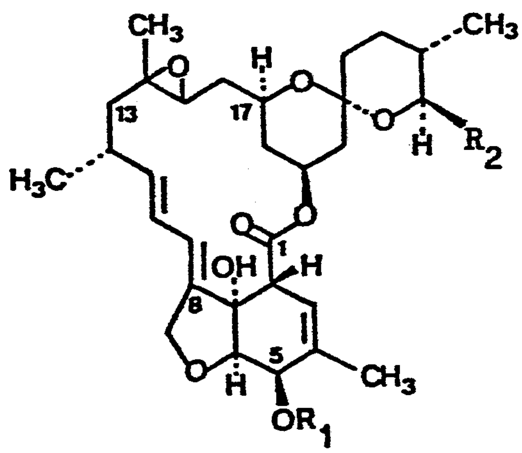

$3^*$

mit Hilfe des Komplex-Reagenzes $[HN_3]_m/Al(Ethyl)_3]_n$, worin m und n unabhängig die Zahl 1 oder 2 oder einen Zahlenwert zwischen 1 und 2 darstellen, in inerten trockenen Lösungsmitteln im Temperaturbereich von -30°C bis +10°C, vorzugsweise -20°C bis -5°C.

Als inerte Lösungsmittel kommen vorzugsweise aliphatische und aromatische Kohlenwasserstoffe wie Benzol, Toluol, Xylol, Petrolether; Ether wie Diethylether, tert.Butylmethylether, Tetrahydrofuran, Dioxan, Anisol in Frage.

Die Reaktion wird vorteilhaft unter Schutzgas wie Stickstoff oder Argon durchgeführt.

Die Reaktion des Epoxids $3^*$ (worin $R_1$ Wasserstoff und $R_2$ Isopropyl bedeutet) mit 14 Aequivalenten $HN_3/Et_3Al$ im molaren Verhältnis 1:0,91 ergibt im Lösungsmittelgemisch Benzol/Toluol bei Raumtemperatur den Azidoalkohol $1^*$ in über 60 % Ausbeute. Die Reaktion des gleichen Epoxids $3^*$ mit 3 Aequivalenten $HN_3/Et_3Al$ (Molverhältnis 1:1,6 liefert in Diethylether neben ca.10 % $1^*$ den Allylalkohol $2^*$ in ca. 50 % Ausbeute (Umsatz ca. 80 %).

Das Komplex-Reagenz $[HN_3]_m/[Al(Ethyl)_3]_n$, worin m und n unabhängig voneinander die Zahl 1 oder 2 oder einen Zahlenwert zwischen 1 und 2 darstellen, wird in inerten Lösungsmitteln aus den Einzelkomponenten $HN_3$ und $Al(C_2H_5)_3$ im obengenannten Temperaturbereich von -30°C bis +10°C hergestellt und ist bei Minustemperaturen einige Zeit stabil. Es soll hier und im folgenden in vereinfachter Schreibweise als $HN_3/Et_3Al$ oder $HN_3/Al(C_2H_5)_3$ wiedergegeben werden. In Gegenwart des Komplexes reagiert eine beliebige Epoxi-Verbindung unter Oxiran-Ringöffnung und unter gleichzeitiger Bildung einer 1-Hydroxy-2-azido-Verbindung und eines substituierten Allylalkohols (=1-Hydroxi-$\Delta^{2,3}$-Verbindung). Beide Reaktionsprodukte lassen sich, wie im Falle der vorliegenden Reaktionsprodukte $1^*$ und $2^*$, durch physikalisch-chemische Trennungsmethoden, z.B. durch fraktionierte Kristallisation oder Chromatographie, voneinander trennen. Unter Chromatographie werden Säulen-, Dickschicht- oder Dünnschicht-Chromatographie an mineralischen Trägermaterialien wie Silicagel oder an organischen Austauscherharzen verstanden.

Stickstoffwasserstoffsäure $HN_3$ lässt sich auch in statu nascendi in den $[HN_3]_m/[Al(Et)_3]_n$-Komplex überführen, indem man im vorgesehenen trockenen Lösungsmittel oder Lösungsmittel-Gemisch Na-Azid suspendiert und daraus mit einer stärkeren Säure, z.B. $H_2SO_4$ (bevorzugt Oleum, um absolut trockene Reaktionsbedingungen zu gewährleisten), $HN_3$ in der

Lösung in Freiheit setzt. $Al(Et)_3$ sollte in der Lösung bereits vorliegen oder kurz danach zugegeben werden. Die zur Reaktion vorgesehene Epoxi-Verbindung kann gleichfalls bereits vorliegen oder zu einem geeigneten Zeitpunkt zur Lösung dosiert werden.

Die in der vorliegenden Erfindung verwendeten Ausgangsverbindungen der Formel 3* lassen sich leicht herstellen durch Epoxidierung der aus der US-PS 3,950,360 bekanntgewordenen und ursprünglich als "Antibiotika B-41-A", später als "Milbemycin-A"-Verbindungen und der aus der US-PS 4,346,171 bekanntgewordenen und als "B-41-D" oder "Milbemycin-D" bezeichneten Verbindungen der Formel sowie der aus der US-PS 4,173,571 bekanntgewordenen 13-Deoxi-Avermectine ($R_2$ = sec.Butyl) der Formel

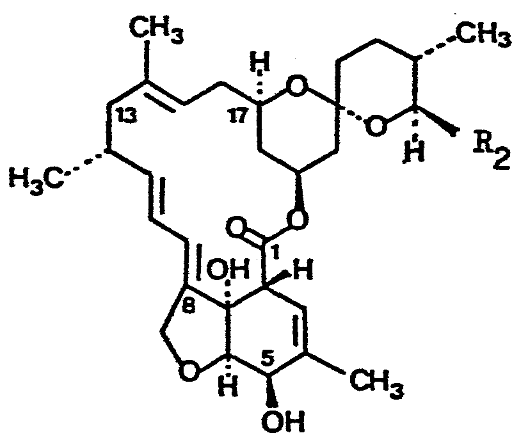

$R_2 = CH_3$ Milbemycin $A_3$
$R_2 = C_2H_5$ Milbemycin $A_4$
$R_2 = isoC_3H_7$ Milbemycin D
$R_2 = sec.C_4H_9$ 13-Deoxi-22,23-dihydro-C-076-Bla-aglycon

Die Epoxidierung wird in einer Lösungsmittelphase im Temperaturbereich von -10°C bis +20°C, vorzugsweise -5°C bis +5°C, durchgeführt.

Zur Epoxidierung kommen Persäuren wie Peressigsäure, Trifluorperessigsäure, Perbenzoesäure, Chlorperbenzoesäure und andere in Frage.

Die neuen als Zwischenprodukte verwendeten Epoxide der Formel $3^*$, worin $R_1$ und $R_2$ die für die Formeln $1^*$ und $2^*$ genannten Bedeutungen haben, sind ein weiterer Gegenstand vorliegender Erfindung, mit Ausnahme des 14,15-Epoxi-Milbemycin $A_3$ ($R_1$ = H; $R_2$ = $CH_3$), des 14,15-Epoxi-Milbemycin $A_4$ ($R_1$ = H; $R_2$ = $C_2H_5$) und des 14,15-Epoxi-Milbemycin-D ($R_1$ = H; $R_2$ = iso$C_3H_7$). Epoxide der Formel $3^*$ sind selbst wertvolle Wirkstoffe zur Bekämpfung von Schädlingen (z.B. Endo-Parasiten und Insekten).

Die Verbindungen der Formeln $1^*$ und $2^*$ eignen sich ausgezeichnet zur Bekämpfung von Schädlingen an Tieren und Pflanzen, darunter tierparasitären Ekto-Parasiten. Zu letzteren zählen unter der Ordnung Acarina insbesondere Schädlinge der Familien Ixodidae, Dermanyssidae, Sarcoptidae, Psoroptidae; die Ordnungen Mallophaga; Siphonaptera, Anoplura (z.B. Familie der Haematopinidae); unter der Ordnung Diptera insbesondere Schädlinge der Familien Muscidae, Calliphoridae, Oesterridae, Tabanidae, Hippoboscidae, Gastrophilidae.

Die Verbindungen $1^*$ und $2^*$ sind auch einsetzbar gegen Hygiene-Schädlinge insbesondere der Ordnungen Diptera mit den Familien Sarcophagidae, Anophilidae, Culicidae; der Ordnung Orthoptera, der Ordung Dictyoptera (z.B. Familie Blattidae) und der Ordnung Hymenoptera (z.B. Familie Formicidae).

Die Verbindungen $1^*$ und $2^*$ besitzen auch nachhaltige Wirksamkeit bei pflanzenparasitären Milben und Insekten. Bei Spinnmilben der Ordnung Acarina sind sie wirksam gegen Eier, Nymphen und Adulte von Tetranychidae (Tetranychus spp. und Panonychus spp.).

Hohe Aktivität besitzen sie bei den saugenden Insekten der Ordnungen Homoptera, insbesondere gegen Schädlinge der Familien Aphididae, Delphacidae, Cicadellidae, Psyllidae, Loccidae, Diaspididae und Eriophyidae (z.B. die Rostmilbe auf Citrusfrüchten); der Ordnungen Hemiptera; Heteroptera und Thysanoptera; sowie bei den pflanzenfressenden Insekten der Ordnungen Lepidoptera; Coleoptera; Diptera und Orthoptera.

Sie sind ebenfalls als Bodeninsektizide gegen Schädlinge im Erdboden geeignet.

Die Verbindungen der Formeln $1^*$ und $2^*$ sind daher gegen alle Entwicklungsstadien saugender und fressender Insekten an Kulturen wie Getreide, Baumwolle, Reis, Mais, Soja, Kartoffeln, Gemüse, Früchten, Tabak, Hopfen, Citrus, Avocados und anderen wirksam.

Die Verbindungen $1^*$ und $2^*$ sind auch wirksam gegen Pflanzen-Nematoden der Arten Meloidogyne, Heterodera, Pratylenchus, Ditylenchus, Radopholus, Rhizoglyphus und andere.

Darüberhinaus sind die Verbindungen $1^*$ und $2^*$ gegen Helminthen wirksam, unter denen die endoparasitären Nematoden die Ursache schwerer Erkrankungen an Säugetieren und Geflügel sein können, z.B. an Schafen, Schweinen, Ziegen, Rindern, Pferden, Eseln, Hunden, Katzen, Meerschweinchen, Ziervögeln. Typische Nematoden dieser Indikation sind: Haemonchus, Trichostrongylus, Ostertagia, Nematodirus, Cooperia, Ascaris, Bunostomum, Oesphagostomum, Chabertia, Trichuris, Strongylus, Trichonema, Dictyocaulus, Cappillaria, Heterakis, Toxocara, Ascaridia, Oxyuris, Ancylostoma, Uncinaria, Toxascaris und Parascaris.

Die Verbindungen $1^*$ oder $2^*$ werden in unveränderter Form oder vorzugsweise zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren wie Versprühen, Vernebeln,

Verstäuben, Verstreuen oder Giessen werden gleich wie die Art der Mittel den angestrebten Zielen und den gegebenen Verhältnissen entsprechend ausgewählt.

Die Verbindungen $1^*$ und $2^*$ werden bei Warmblütern in Aufwandmengen von 0,01 bis 50 mg/kg Körpergewicht angewendet, über geschlossenen Kultur-Anbauflächen, in Pferchen, Stallungen oder sonstigen Räumen in Mengen von 10 g bis 1000 g pro Hektar.

Die Formulierungen, d.h. die den Wirkstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Aether und Ester, wie Aethanol, Aethylenglykol, Aethylenglykolmonomethyl- oder -äthyläther, Ketone wie Cyclohexanon, stark polare Lösungsmittel wie N-Methyl-2-pyrrolidon, Dimethylsulfoxid oder Dimethylformamid, sowie gegebenenfalls epoxydierte Pflanzenöle wie epoxydiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen,

wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes nichtionogene, kation- und/oder anionaktive Tenside mit gutem Emulgier-, Dispergier- und Netzeigenschaften in Betracht. Unter Tensiden sind auch Tensidgemische zu verstehen.

Geeignete anionische Tenside können sowohl sog. wasserlösliche Seifen wie wasserlösliche synthetische oberflächenaktive Verbindungen sein.

Als Seifen eignen sich die Alkali-, Erdalkali- oder gegebenenfalls substituierten Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Talgöl gewonnen werden können. Ferner sind auch die Fettsäuremethyl-taurinsalze zu erwähnen.

Häufiger werden jedoch sog. synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches.

Hierher gehören auch die Salze der Schwefelsäureester und Sulfonsäuren von Fettalkohol-Aethylenoxid-Addukten. Die sulfonierten Benzimidazolderivate enthalten vorzugsweise 2-Sulfonsäuregruppen und einen Fettsäurerest mit 8-22 C-Atomen. Alkylarylsulfonate sind z.B. die Na-, Ca- oder Triäthanolaminsalze der Dodecylbenzolsulfonsäure, der Dibutylnaphthalinsulfonsäure, oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Aethylenoxid-Adduktes oder Phospholipide in Frage.
Die in der Formulierungstechnik gebräuchlichen Tenside sind u.a. in folgenden Publikationen beschrieben:

"Mc Cutcheon's Detergents and Emulsifiers Annual" MC Publishing Corp., Ringwood, New Jersey, 1982.

Die pestiziden Zubereitungen enthalten in der Regel 0,01 bis 95 %, insbesondere 0,1 bis 80 %, Wirkstoff, 5 bis 99,99 % eines festen oder flüssigen Zusatzstoffes und 0 bis 25 %, insbesondere 0,1 bis 25 %, eines Tensides.

Während als Handelsware eher konzentrierte Mittel bevorzugt werden, verwendet der Endverbraucher in der Regel verdünnte Mittel mit 1-10'000 ppm Wirkstoffgehalt.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Die Verbindungen der Formel $1^*$ und besonders die der Formel $2^*$ stellen auch vielseitig reaktionsfähige Produkte zur Gewinnung weiterer Milbemycin-Derivate dar.

Herstellungsbeispiele:

1. Herstellung von wasserfreiem $HN_3$

1.1. In Benzol. Die Herstellung erfolgte nach der Vorschrift von H. Wolf, Org. Reactions 3, 307 (1946). Die dabei erhaltene Lösung von $HN_3$ in Benzol wurde zweimal über wasserfreiem $Na_2SO_4$ (30 Min. auf einer Flamme erhitzt und im Exsikkator abgekühlt) getrocknet und bei 0°C ⟶ 5°C durch Watte abfiltriert. Die Lösung wurde bei 4°C im Kühlschrank aufbewahrt.

1.2. In Aether. Die Herstellung erfolgte analog zur obigen Vorschrift, ausser dass bei der Zugabe der Schwefelsäure die Reaktionstemperatur zwischen -20°C und -10°C gehalten wurde; nach beendeter $H_2SO_4$ Zugabe wurde das Reaktionsgemisch auf -5°C erwärmt.

2. Herstellung von 14,15-Epoxi-Milbemycin D (Formel 3*)

Zu einer Lösung von 550 mg Milbemycin D in 5 ml Dichlormethan wurde unter Eiskühlung eine Lösung von 170 mg Chlorperbenzoesäure in 5 ml Dichlormethan gegeben. Nach 1-stündigem Rühren bei 0° bis 5°C wurden nochmals 170 mg des Oxidationsmittels hinzugefügt und weitere 30 min. gerührt. Nach beendeter Reaktion wurde die Lösung in eine eisgekühlte Lösung von Natriumsulfit gegossen und mit Essigsäureethylester extrahiert. Die vereinigten Extrakte wurden einmal mit Wasser gewaschen, getrocknet und im Vakuum eingedampft. Das Rohprodukt wurde durch Chromatographierung über eine Silicagel-Säule (Elutionsmittel n-Hexan/Essigsäureethylester 20:15) gereinigt. Es wurden 450 mg 14,15-Epoxi-Milbemycin D als amorphe, weisse Substanz erhalten.

3. Herstellung von 14-Azido-15-hydroxy-Milbemycin-D (Formel 1*)

In einen mit einem Magnetrührer, einem Thermometer und einer Serumkappe versehenen trockenen 50 ml Zweihalskolben wurden unter Argon 5 ml abs. Toluol gegeben. Bei 0°C und unter Rühren wurden mit einer Spritze 1,7 ml (1,39 g, 12,2 mmol) Triethylaluminium ($Et_3Al$) zugegeben. Nach 5 Min. wurden bei -20°C 10,7 ml (0,58 g, 13,4 mmol) einer 5,72 proz.-Lösung von $HN_3$ in Benzol langsam unter starkem Rühren zugegeben; die Temperatur des Reaktionsgemisches wurde zwischen -20°C und -10°C gehalten (die Reaktion ist stark exotherm). Nach weiteren 5 Min. Rühren bei -10°C wurde das Reaktionsgemisch mit einer Spritze rasch und unter starkem Rühren zu einer auf -10°C gekühlten Lösung von 0,50 g (0,88 mmol) 14,15-Epoxi-Milbemycin D in 5 ml abs. Toluol gegeben (unter Argon). Das Reaktionsgemisch wurde dann 2 Std. bei Raumtemperatur gerührt.

Zur Aufarbeitung wurden 10 ml Methylenchlorid zugegeben, das Gemisch wurde 10 Min. im offenen Kolben gerührt und dann in 1 Liter 2 proz. wässerige HCl-Lösung (oder 250 ml 4 proz. HCl) gegossen. Es wurde 4 mal mit 50 ml $CH_2Cl_2$ extrahiert, die organische Phase mit gesättigter NaCl-Lösung gewaschen und über $NaSO_4$ getrocknet. Die Chromatographie an 40 g Kieselgel ($CH_2Cl_2$/Aceton 25:1) ergab 328 mg (61 %) 14-Azido-15-hydroxi-Milbemycin D (1) als farblose Kristalle, Smp. 189°C (aus Methanol).

4. Herstellung von 15-Hydroxi-$\Delta^{13,14}$-Milbemycin D (Formel 2*)

Analog zu Vorschrift 3 wurden bei -20°C zu einer Lösung von 2,1 ml (1,75 g, 15,3 mmol) Triethylaluminium in 8,5 ml abs. Diethylether 8.4 ml (0,41 g, 9,53 mmol) einer 6,96 proz. Lösung von $HN_3$ in Diethyl-Ether gegeben, die dann bei -10°C unter stark exothermer

Reaktion zu 1,8 g (3,15 mmol) 14,15-Epoxi-Milbemycin D (in Substanz) gegeben wurde. Nach 1 Stunde bei Raumtemperatur wurden 4 ml absoluter Ether zugegeben und das gallertige Reaktionsgemisch wurde kräftig gerührt. Nach 4 Stunden wurde wie in Vorschrift 2 aufgearbeitet und die Chromatographie an 70 g Kieselgel ($CH_2Cl_2$/Aceton 10:1) ergab 200 mg (10 %) 14-Azido-15-hydroxi-Milbemycin D (1) und 820 mg (45 %) 15-Hydroxy-$\Delta^{13,14}$-Milbemycin D (2), Smp.: 151°C - 153°C (aus Methanol).

$^1$H-NMR (300 MHz . $CDCl_3$.TMS) der letztgenannten Verbindung:
1,58 (breites s)($C_{14}CH_3$); 1,88 (breites s)($C_4CH_3$);
4,06 (dd)($J_1$=11,1; $J_2$ = 4,6)($C_{15}H$); 5,20(d)(J = 10,3)($C_{13}H$).

5. Herstellung von 5-O-t-Butyldimethylsilyl-14,15-epoxi-Milbemycin D

Eine Lösung von 2,21 g (3,86 mmol) 14,15-Epoxi-Milbemycin D ($R_2$ = iso$C_3H_7$), 757 mg (5,02 mmol) t-Butyl dimethylchlorsilan und 342 mg (5,02 mmol) Imidazol in 4 ml DMF wurde 90 min. bei Raumtemperatur gerührt. Anschliessend wurden 80 ml Diethylether zugegeben, und das Gemisch wurde über 20 g Kieselgel filtriert und eingeengt. Es wurden 2,65 g (100%) 5-O-t-Butyldimethylsilyl-14,15-epoxi-Milbemycin D erhalten.

$^1$H-NMR (300MHz. $CDCl_3$):
0,12 (s) $(CH_3)_2$Si-O-;
0,92 (s) (t.-$C_4H_9$)Si-O-;
1,23 (breites s)($C_{14}CH_3$);
2,56 (d; J = 9) ($C_{15}H$).

Aehnlich lässt sich durch Reaktion mit Trimethylsilyl-trifluormethansulfonat das entsprechende 5-O-Trimethylsilyl-14,15-epoxi-Milbemycin D herstellen, Smp. 92-97°C.

6. Herstellung von 5-O-t-Butyldimethylsilyl-15-hydroxi-$\Delta^{13,14}$-Milbemycin D(Formel 2*)

Eine Lösung des $HN_3$/$Et_3$Al - Komplex-Reagenz (hergestellt aus einer Lösung von 4,97 ml Triethylaluminium in 7 ml abs. Tetrahydrofuran (THF) und 9,15 ml einer 2,39 M Lösung von $HN_3$ (21.9 mmol) in abs.

Diethylether wurde unter Argon zu einer Lösung von 5.0 g (7,29 mmol) 5-O-Butyldimethylsilyl-14,15-epoxi-Milbemycin D in ca. 20 ml abs. THF gegeben, und das Gemisch wurde 16 Std. unter Rückfluss erhitzt. Anschliessend wurden bei Raumtemperatur 250 ml Ether, 2 ml Methanol und schliesslich ein Gemisch von 10 g $Na_2SO_4 \cdot 10\ H_2O$ und 10 g Celite zugegeben. Das Gemisch wurde filtriert, eingeengt, und die Chromatographie des Rohproduktes an 160 g Kieselgel (0 - 30% Essigsäureethylester in Hexan) ergab 2,37 g (47%) 5-O-t-Butyldimethylsilyl-15-hydroxi-$\Delta^{13,14}$-Milbemycin D.

$^1H$-NMR (300 MHz, $CDCl_3$):
1,59 (d; J = 1) ($C_{14}CH_3$);
4,06 (dd; $J_1$ = 11; $J_2$ = 4) ($C_{15}H$);
5,15 (d; J = 8) ($C_{13}H$).

Daneben wurden 109 mg (2%) 13β-Azido-5-O-t-butyldimethylsilyl-Milbemycin D gewonnen.

$^1H$-NMR (300 MHz, $CDCl_3$):
1,57 (breites s)($C_{14}CH_3$);
3,56 (d; J = 9) ($C_{13}H$);
IR: 2095 $cm^{-1}$ ($N_3$).

Eine Lösung von 100 mg (0.146 mmol) dieses Produkts in 2 ml 1proz. p-Toluolsulfonsäure (in Methanol) wurde 40 min. bei Raumtemperatur gerührt, dann mit 20 ml Ether verdünnt und durch Kieselgel filtriert. Das Rohprodukt wurde zuerst in Ethanol und dann in Benzol aufgenommen, und das Lösungsmittel wurde jeweils im Vakuum abgedampft. Es wurden 53 mg (63%) 13β-Azido-Milbemycin D erhalten.

$^1H$-NMR (300 MHz, $CDCl_3$):
1,57 (br. s) ($C_{14}CH_3$);
3,54 (d; I = 11) ($C_{13}H$);

Massenspektrum m/e : 597 ($M^+$), 569, 151.

7. Herstellung von 14,15-Epoxi-Milbemycin $A_4$ (Formel 3*)

Zu einer Lösung von 5,7 g (10,5 mmol) Milbemycin $A_4$ in 140 ml Dichlormethan und 120 ml 0,5 M $NaHCO_3$-Lösung wurde bei Raumtemperatur eine Lösung von 2,43 g (14,08 mmol) m-Chlorobenzopersäure in 70 ml Dichlormethan tropfenweise zugegeben. Das Gemisch wurde 1 Std. bei Raumtemperatur kräftig gerührt und dann mit 300 ml Dichlormethan verdünnt. Die organische Phase wurde mit wässriger $NaHCO_3$-Lösung gewaschen, mit $Na_2SO_4$ getrocknet und eingeengt. Es wurden 5,7 g Epoxid als Rohprodukt erhalten.

8. Herstellung von 5-O-t-Butyldimethylsilyl-14,15-epoxi-Milbemycin $A_4$ (Formel 3*)

5,7 g 14,15-Epoxi-Milbemycin $A_4$ wurden in 10 ml trockenem Dimethylformamid (DMF) gelöst. Bei Raumtemperatur wurden 0,63 g (9,16 mmol) Imidazol und 1,4 g (9,34 mmol) t-Butyldimethylchlorsilan zugegeben. Das Gemisch wurde 1 Std. bei Raumtemperatur gerührt und an 150 g Kieselgel chromatographiert (Hexan/Ether 4:1), wobei 2,84 g (40% d. Th. Ausbeute, bezogen auf Milbemycin $A_4$) des silylierten Epoxi-Derivats erhalten wurden.

9. Herstellung von 5-O-t-Butyldimethylsilyl-15-hydroxi-$\Delta^{13,14}$-Milbemycin $A_4$ (Formel 2*)

Nach der Verfahrensweise des Beispiels 3 wurde das Komplex-Reagenz $HN_3$/Al (Ethyl)$_3$ wie folgt hergestellt:
2,8 ml (12,2 mmol) $Al(C_2H_5)_3$ in 4 ml abs. THF wurden unter Argon bei ca. -20°C langsam mit 5,28 ml (20,4 mmol) einer 10%igen Lösung von $HN_3$ in abs. Diethylether versetzt. Zu dieser Lösung wurde unter Argon eine Lösung von 2,84 g (4,25 mmol) der im Beispiel 8 erhaltenen Verbindung gegeben, und das so erhaltene Gemisch wurde 4 Std. unter Rückfluss erhitzt. Bei Raumtemperatur wurden 500 ml Ether und 10 g

$Na_2SO_4 \cdot 10H_2O$ und 10 g Celite zugegeben, und das Gemisch wurde filtriert und eingeengt. Die Chromatographie des Rohproduktes an 100 g Kieselgel (Hexan/Diethylether 7:2) ergab 1,72 g (60% d.Th) der Titel-Verbindung.

$^1H$-NMR (300 MHz, $CDCl_3$):
1,59 (br. s) ($C_{14}CH_3$);
4,05 (br. s) ($C_{15}H$);
5,15 (d; J = 6) ($C_{13}H$).

Daneben wurden 0,1 g 13β-Azido-5-O-t-butyldimethylsilyl-Milbemycin $A_4$ erhalten.

10. Herstellung von 15-Hydroxi-$\Delta^{13,14}$-Milbemycin $A_4$ (Formel 2*)

Die Hydrolyse von 5 mg der im Beispiel 9 genannten Titelverbindung mit 1 ml einer 1%ig.-Lösung von p-Toluolsulfonsäure in Methanol und die Aufarbeitung in Diethylether mit 5%ig. Na-Hydrogencarbonat-Lösung ergab 4,5 mg der Titel-Verbindung.

$^1H$-NMR (300 MHz. $CDCl_3$, TMS);
0,98 (t) (J = 7) ($C_{25}CH_2$-$CH_3$); 1,59 (br.s.) ($C_{14}CH_3$);
1,87 (br. s) ($C_4CH_3$); 4,07 (ddd)($J_1$=10,8; $J_2$=3, 1; $J_3$=1,2)($C_{15}H$);
5,15(d) (J = 10,4)($C_{13}H$).

11. Herstellung von 14,15-Epoxi-Milbemycin $A_3$ ($R_2$ = $CH_3$)

Nach der Vorschrift des Beispiels 2 werden aus 220 mg Milbemycin $A_3$ in 5 ml Dichlormethan und 320 mg Benzopersäure in 5 ml Dichlormethan bei -2°C bis +5°C während 1,5 Std. und Reinigung über eine Silicagel-Säule 190 mg 14,15-Epoxi-Milbemycin $A_3$ gewonnen.

12. Herstellung von 5-O-Methyldiphenylsilyl-14,15-epoxi-Milbemycin $A_3$

Nach der Vorschrift des Beispiels 5 werden aus 190 mg 14,15-Epoxi-Milbemycin $A_3$ und 120 mg Diphenylmethylchlorsilan in Gegenwart von Imidazol 217 mg der Titel-Verbindung erhalten.

13. Herstellung von 5-O-Methyldiphenylsilyl-15-hydroxi-$\Delta^{13,14}$-Milbemycin $A_3$ (Formel 2*)

Analog zur Epoxid-Spaltung des Beispiels 6 werden aus 210 mg 5-O-Methyldiphenylsilyl-14,15-epoxi-Milbemycin $A_3$ in absolutem Diethylether

mit Hilfe des Komplex-Reagenz $HN_3/Et_3Al$ unter Argon nach anschliessender Reinigung 203 mg der Titelverbindung erhalten.

$^1$H-NMR (300 HMz, $CDCl_3$):

1,58 (br. s)($C_{14}CH_3$);

4,05 (br. s)($C_{15}H$);

5,15 (d; J=6)($C_{13}H$).

14. Herstellung von 15-Hydroxi-$\Delta^{13,14}$-Milbemycin $A_3$ und 14-Azido-15-hydroxi-Milbemycin $A_3$

Analog zu Beispiel 2 wird das Reagenz $HN_3/Al(C_2H_5)_3$ frisch hergestellt und bei -10°C zu einer Lösung von 830 mg (3,05 mmol) 14,15-Epoxi-Milbemycin $A_3$ in 7 ml trockenem Diethylether getropft. Nach der Aufarbeitung werden 385 mg 15-Hydroxi-$\Delta^{13,14}$-Milbemycin $A_3$ und 92 mg 14-Azido-15-hydroxi-Milbemycin $A_3$ erhalten.

15. Herstellung von 13-Deoxi-14,15-epoxi-22,23-dihydro-avermectin-Bla-aglykon ($R_2$ = sec.$C_4H_9$) (Formel 3*)

Analog zu Beispiel 7 erhält man aus 520 mg 13-Deoxi-22,23-dihydro-avermectin-Bla-aglykon [Tetrahedron Letters, Vol. 24, No. 48, pp 5333-5336 (1983)] und 210 mg m-Chlorbenzopersäure in 20 ml Dichlormethan 510 mg der Titelverbindung.

16. Herstellung von 5-O-t.Butyldimethylsilyl-13-deoxi-14,15-epoxi-22,23-dihydro-avermectin-Bla-aglykon (Formel 3*)

Analog zu Beispiel 8 erhält man aus 220 mg der Titelverbindung von Beispiel 15 und 55 mg tert.Butyldimethylchlorsilan in Gegenwart von 25 mg Imidazol in 5 ml trockenem DMF 108 mg der Titelverbindung.

17. Herstellung von 13-Deoxi-15-hydroxi-$\Delta^{13,14}$-22,23-dihydro-avermectin-Bla-aglykon (Formel 2*)

Analog zu Beispiel 4 erhält man aus 220 mg der Titelverbindung von Beispiel 15 mit dem Komplex-Reagenz, bestehend aus 320 mg $Al(C_2H_5)_3$ und 110 mg einer 6,96%ig-Lösung von $HN_3$ in total 16 ml trockenem Diethylether 112 mg der Titelverbindung. Daneben werden 61 mg 13-Deoxi-14-azido-15-hydroxi-22,23-dihydro-avermectin-Bla-aglykon (Formel 1*) erhalten.

Beispiele für weitere erfindungsgemässe Zwischenprodukte der Formel 3*

5-O-Trimethylsilyl-14,15-epoxi-Milbemycin $A_3$
5-O-tris(tert.Butyl)silyl-14,15-epoxi-Milbemycin $A_3$
5-O-Diphenyl-tert.butylsilyl-14,15-epoxi-Milbemycin $A_3$,
5-O-bis(Isopropyl)methylsilyl-14,15-epoxi-Milbemycin $A_3$,
5-O-bis(Allyl)(tert.butyl)silyl-14,15-epoxi-Milbemycin $A_3$,
5-O-tert.Butyldimethylsilyl-14,15-epoxi-Milbemycin $A_3$,
5-O-tris(Benzyl)silyl-14,15-epoxi-Milbemycin $A_3$ ;

5-O-Trimethylsilyl-14,15-epoxi-Milbemycin $A_4$,
5-O-tris(tert.Butyl)silyl-14,15-epoxi-Milbemycin $A_4$
5-O-Diphenyl-tert-butylsilyl-14,15-epoxi-Milbemycin $A_4$,
5-O-bis(Isopropyl)methylsilyl-14,15-epoxi-Milbemycin $A_4$,
5-O-bis(Allyl)(tert.butyl)silyl-14,15-epoxi-Milbemycin $A_4$,
5-O-tert.Butyldiethylsilyl -14,15-epoxi-Milbemycin $A_4$,
5-O-Methylphenylsilyl-14,15-epoxi-Milbemycin $A_4$,
5-O-tris(Benzyl)silyl-14,15-epoxi-Milbemycin $A_4$,

5-O-Tris(ethyl)silyl-14,15-epoxi-Milbemycin-D,
5-O-tris(tert.Butyl)silyl-14,15-epoxi-Milbemycin-D,
5-O-Diphenyl-tert.butylsilyl-14,15-epoxi-Milbemycin-D,
5-O-bis(Isopropyl)methylsilyl-14,15-epoxi-Milbemycin-D,
5-O-Methyldiphenylsilyl-14,15-epoxi-Milbemycin-D,
5-O-tris(Benzyl)silyl-14,15-epoxi-Milbemycin-D,

sowie Verbindungen der Avermectin-Reihe ($R_2$ = sec.$C_4H_9$)

5-O-Tris(ethyl)-silyl-,

5-O-Trimethylsilyl-,
5-O-tris(tert.Butyl)silyl-,
5-O-Diphenyl-tert.butylsilyl-,
5-O-bis(Isopropyl)methylsilyl-,
5-O-bis(Allyl)(tert.butyl)silyl-,
5-O-Methyldiphenylsilyl-,
5-O-tris-(Benzyl)silyl-,
5-O-tris(Isopropyl)silyl-, und
5-O-bis(Isopropyl)methyl-13-deoxi-14,15-epoxi-22,23-dihydro-avermectin-Bla-aglycon.

5-O-Acetyl-14,15-epoxi-Milbemycin $A_3$
5-O-Propionyl-14,15-epoxi-Milbemycin $A_3$
5-O-Pivalyl-14,15-epoxi-Milbemycin $A_3$
5-O-Benzoyl-14,15-epoxi-Milbemycin $A_3$
5-O-Mesyl-14,15-epoxi-Milbemycin $A_3$
5-O-Tosyl-14,15-epoxi-Milbemycin $A_3$

5-O-Acetyl-14,15-epoxi-Milbemycin $A_4$
5-O-Propionyl-14,15-epoxi-Milbemycin $A_4$
5-O-Pivalyl-14,15-epoxi-Milbemycin $A_4$
5-O-Benzoyl-14,15-epoxi-Milbemycin $A_4$
5-O-p-Chlorbenzoyl-14,15-epoxi-Milbemycin $A_4$
5-O-Mesyl-14,15-epoxi-Milbemycin $A_4$
5-O-Ethylsulfonyl-14,15-epoxi-Milbemycin $A_4$
5-O-Benzolsulfonyl-14,15-epoxi -Milbemycin $A_4$
5-O-Tosyl-14,15-epoxi-Milbemycin $A_4$
5-O-p-Chlorbenzolsulfonyl-14,15-epoxi-Milbemycin $A_4$
5-O-o-Nitrobenzolsulfonyl-14,15-epoxi-Milbemycin $A_4$

5-O-Acetyl-14,15-epoxi-Milbemycin D
5-O-Propionyl-14,15-epoxi-Milbemycin D
5-O-Pivalyl-14,15-epoxi-Milbemycin D
5-O-Benzoyl-14,15-epoxi-Milbemycin D

5-O-p-Chlorbenzoyl-14,15-epoxi-Milbemycin D
5-O-Mesyl-14,15-epoxi-Milbemycin D
5-O-Ethylsulfonyl-14,15-epoxi-Milbemycin D
5-O-Benzolsulfonyl-14,15-epoxi—Milbemycin D
5-O-Tosyl-14,15-epoxi-Milbemycin D
5-O-p-Chlorbenzolsulfonyl-14,15-epoxi-Milbemycin D
5-O-o-Nitrobenzolsulfonyl-14,15-epoxi-Milbemycin D

5-O-Acetyl-13-deoxi-14,15-epoxi-22,23-dihydroavermectin-B-1a-aglykon
5-O-Pivalyl-13-deoxi-14,15-epoxi-22,23-dihydroavermectin-B-1a-aglykon
5-O-Benzoyl-13-deoxi-14,15-epoxi-22,23-dihydroavermectin-B-1a-aglykon
5-O-Mesyl-13-deoxi-14,15-epoxi-22,23-dihydroavermectin-B-1a-aglykon
5-O-Tosyl-13-deoxi-14,15-epoxi-22,23-dihydroavermectin-B-1a-aglykon.

<u>Beispiele für weitere erfindungsgemässe Verbindungen der Formel 1*</u>

5-O-Trimethylsilyl-14-azido-15-hydroxi-Milbemycin $A_3$
5-O-Diphenyl-tert.butylsilyl-14-azido-15-hydroxi-Milbemycin $A_3$
5-O-tert.Butyldimethylsilyl-14-azido-15-hydroxi-Milbemycin $A_3$
5-O-Trimethylsilyl-14-azio-15-hydroxy-Milbemycin $A_4$
5-O-tris(tert.Butyl)silyl-14-azido-15-hydroxy-Milbemycin $A_4$
5-O-Diphenyl-tert.butylsilyl-14-azido-15-hydroxi-Milbemycin $A_4$
5-O-tert.Butyldimethylsilyl-14-azido-15-hydroxi-Milbemycin $A_4$
5-O-tert.Butyldiethylsilyl-14-azido-15-hydroxi-Milbemycin $A_4$
5-O-tris(Ethyl)silyl-14-azido-15-hydroxi-Milbemycin D
5-O-tris(tert.Butyl)silyl-14-azio-15-hydroxi-Milbemycin D
5-O-Methyldiphenylsilyl-14-azido-15-hydroxi-Milbemycin D
5-O-tris(Benzyl)silyl-14-azido-15-hydroxi-Milbemycin D
5-O-tris(Ethyl)silyl-13-deoxi-14-azido-15-hydroxi-22,23-dihydro-avermectin -Bla-aglykon
5-O-Acetyl-14-azido-15-hydroxi-Milbemycin $A_3$
5-O-Acetyl-14-azido-15-hydroxi-Milbemycin $A_4$
5-O-Acetyl-14-azido-15-hydroxi-Milbemycin D
5-O-Acetyl-13-deoxi-14-azido-15-hydroxi-22,23-dihydro-avermectin-Bla-aglykon.

Beispiele für weitere erfindungsgemässe Verbindungen der Formel 2*

5-O-Trimethylsilyl-15-hydroxi-$\Delta^{13,14}$-Milbemycin $A_3$

5-O-Diphenyl-tert.butylsilyl-15-hydroxi-$\Delta^{13,14}$-Milbemycin $A_3$

5-O-tert.Butyldimethylsilyl-15-hydroxi-$\Delta^{13,14}$-Milbemycin $A_3$

5-O-Trimethylsilyl-15-hydroxi-$\Delta^{13,14}$-Milbemycin $A_4$

5-O-tris(tert.Butyl)silyl-15-hydroxi-$\Delta^{13,14}$-Milbemycin $A_4$

5-O-Diphenyl-tert.butylsilyl-15-hydroxi-$\Delta^{13,14}$-Milbemycin $A_4$

5-O-tert.Butyldimethylsilyl-15-hydroxi-$\Delta^{13,14}$-Milbemycin $A_4$

5-O-tert.Butyldiethylsilyl-15-hydroxy-$\Delta^{14,14}$-Milbemycin $A_4$

5-O-tris(Ethyl)silyl-15-hydroxi-$\Delta^{13,14}$-Milbemycin D

5-O-tris(tert.Butyl)silyl-15-hydroxi-$\Delta^{13,14}$-Milbemycin D

5-O-Methyldiphenylsilyl-15-hydroxi-$\Delta^{13,14}$-Milbemycin D

5-O-tris(Benzyl)silyl-15-hydroxy-$\Delta^{13,14}$-Milbemycin D

5-O-tris(Ethyl)silyl-13-deoxi-$\Delta^{13,14}$-15-hydroxy-22,23-dihydro-avermectin-B1a-aglykon

5-O-Acetyl-15-hydroxi-$\Delta^{13,14}$-Milbemycin $A_3$

5-O-Acetyl-15-hydroxy-$\Delta^{13,14}$-Milbemycin $A_4$

5-O-Acetyl-15-hydroxi-$\Delta^{13,14}$-Milbemycin D

5-O-Acetyl-13-deoxi-$\Delta^{13,14}$-15-hydroxi-22,23-dihydro-avermectin-B1a-aglykon.

Formulierungsbeispiele für Wirkstoffe

(% = Gewichtsprozent)

Spritzpulver

| | a) | b) | c) |
|---|---|---|---|
| Wirkstoff 1* oder 2* | 25% | 50% | 75% |
| Na-Ligninsulfonat | 5% | 5% | - |
| Na-Laurylsulfat | 3% | - | 5% |
| Na-Diisobutylnaphthalinsulfonat | - | 6% | 10% |
| Octylphenolpolyäthylenglykoläther (7-8 Mol AeO) | - | 2% | - |
| Hochdisperse Kiselsäure | 5% | 10% | 10% |
| Kaolin | 62% | 27% | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

Emulsions-Konzentrat

| | |
|---|---|
| Wirkstoff 1* oder 2* | 10% |
| Octylphenolpolyäthylenglykoläther (4-5 Mol AeO) | 3% |
| Ca-Dodecylbenzolsulfonat | 3% |
| Ricinusölpolyglykoläther (36 Mol AeO) | 4% |
| Cyclohexanon | 30% |
| Xylolgemisch | 50% |

Aus diesem Konzentrat können durch Verdünnen mit Wasser Emulsionen jeder gewünschten Konzentration hergestellt werden.

| Stäubemittel | a) | b) |
|---|---|---|
| Wirkstoff 1* oder 2* | 5% | 8% |
| Talkum | 95% | - |
| Kaolin | - | 92% |

Man erhält anwendungsfertige Stäubemittel, indem der Wirkstoff mit dem Träger vermischt und auf einer geeigneten Mühle vermahlen wird.

Extruder Granulat

| | |
|---|---|
| Wirkstoff 1* oder 2* | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschliessend im Luftstrom getrocknet.

Anstatt eines Wirkstoffs 1* oder 2* kann auch eines der biologisch aktiven Zwischenprodukte der Formel 3* auf eine der vorstehend genannten Arten formuliert werden.

Falls die Verbindungen 1* oder 2* bzw. entsprechende Mittel zur Bekämpfung von Ektoparasiten an Haus- und Nutztieren, wie Rindern, Schafen, Ziegen, Katzen und Hunden, verwendet werden, können sie den Tieren sowohl als Einzeldosis wie auch wiederholt mittels der Pour-on-Methode auf den Körper appliziert werden.

## Biologische Beispiele

### B-1. Insektizide Frassgift-Wirkung bei Spodoptera littoralis

Eingetopfte Baumwollpflanzen im 5-Blatt-Stadium werden mit einer acetonisch/wässrigen Versuchslösung besprüht, die 3; 12,5 oder 50 ppm der zu prüfenden Verbindung enthält.

Nach dem Antrocknen des Belags werden die Pflanzen mit ca. 30 Larven ($L_1$-Stadium) von Spodoptera littoralis besetzt. Pro Versuchsverbindung und pro Test-Spezies verwendet man zwei Pflanzen. Der Versuch wird bei ca. 24°C und 60 % relativer Luftfeuchigkeit durchgeführt. Auswertungen und Zwischenauswertungen auf moribunde Tiere, Wachstum der Larven und Frassschäden erfolgen nach 24 Stunden; 48 Stunden und 72 Stunden.

Verbindungen der Formeln 2* oder 3* erzielen bei einer Konzentration von 12,5 ppm eine vollständige Abtötung nach 24 Stunden.

B-2. Wirkung gegen pflanzenschädigende Akariden

OP-sensible Tetranychus urticae

Die Primärblätter von Bohnenpflanzen (Phaseolus vulgaris) werden 16 Std. vor dem Versuch mit einem durch T. urticae infestierten, aus einer Massenzucht stammenden Blattstück belegt. Die so mit allen Milben-Stadien infestierten Pflanzen werden nach Entfernung des Blattstücks mit einer Versuchslösung bis zur Tropfnässe besprüht, die wahlweise 0,4 ppm oder 1,6 ppm der zu prüfenden Verbindung enthält. Die Temperatur in der Gewächshauskabine beträgt ca. 25° C.

Nach sieben Tagen wird unter dem Binokular auf Prozentsatz mobiler Stadien (Adulte und Nymphen) und auf vorhandene Eier ausgewertet.

Die Verbindungen der Formeln 1* und 2* erzielen bei einer Wirkstoffkonzentration von 1,6 ppm vollständige Abtötung oder 95% Wirksamkeit.

B-3. Wirkung gegen $L_1$-Larven von Lucilia sericata

1 ml einer wässrigen Suspension der zu prüfenden Aktivsubstanz werden so mit 3 ml eines speziellen Larvenzuchtmediums bei ca. 50°C vermischt, dass ein Homogenisat von wahlweise 100 ppm bzw. 10 ppm Wirkstoffgehalt entsteht. In jeder Reagenzglas-Probe werden ca. 30 Lucilia-Larven ($L_1$) eingesetzt. Nach 4 Tagen wird die Mortalitätsrate bestimmt. Die Verbindungen der Formeln 1*, 2* oder 3* erzielen mit 100 ppm Aufwandmenge eine Wirkung von über 90%.

Patentansprüche:

1. 14-Azido-15-hydroxi-Milbemycin-Derivate der Formel 1* und 15-Hydroxi-$\Delta^{13,14}$-Milbemycin-Derivate der Formel 2*

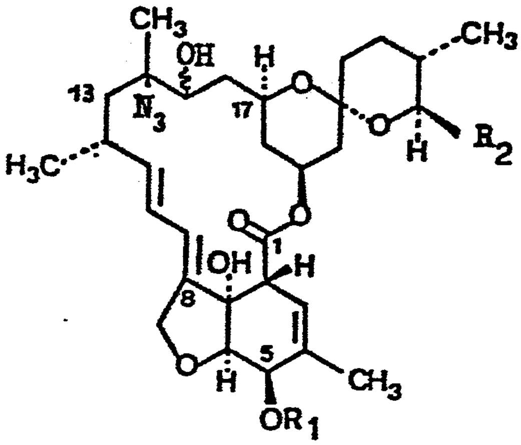

1*

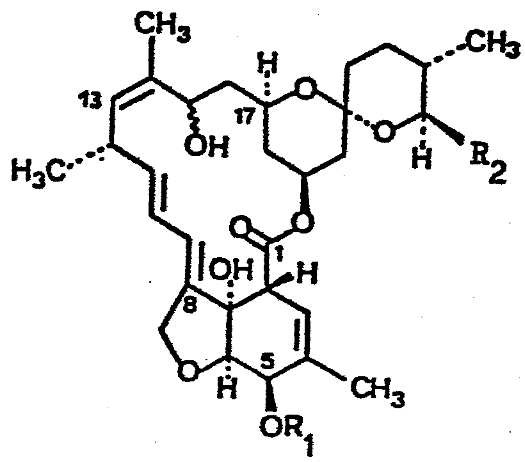

2*

worin $R_1$ Wasserstoff oder eine Silyl- oder Acylgruppe und $R_2$ Methyl, Ethyl, Isopropyl oder sek.Butyl bedeuten.

2. Verbindungen gemäss Anspruch 1, worin $R_1$ Wasserstoff und $R_2$ Isopropyl bedeuten.

3. Verbindungen gemäss Anspruch 1, worin $R_1$ Wasserstoff und $R_2$ Methyl, Ethyl oder sek.Butyl bedeuten.

4. Verbindungen gemäss Anspruch 1 der Formel 2*.

5. Verbindungen gemäss Anspruch 1 der Formeln 1* und 2*, worin eine Acylgruppe $R_1$ einen $R_3CO$-Rest oder einen $R_4SO_2$-Rest bedeutet, in welchem $R_3$ einen unsubstituierten oder halogenierten $C_1$-$C_6$-aliphatischen Rest oder eine unsubstituierte oder durch $C_1$-$C_4$-Alkyl oder Halogen substituierte Phenylgruppe darstellt, und $R_4$ einen $C_1$-$C_4$-Alkylrest oder einen unsubstituierten oder durch Methyl, Chlor oder Nitro substituierten Phenylrest darstellt.

6. Verbindungen gemäss Anspruch 1 der Formeln 1* und 2*, worin $R_1$ Wasserstoff oder die Silylgruppe

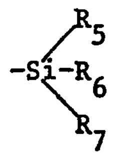

darstellt, in der $R_5$ Methyl, Ethyl, Propyl, Isopropyl, tert.Butyl und $R_6$ und $R_7$ unabhängig Methyl, Ethyl, Isopropyl, tert.Butyl, Phenyl oder Benzyl bedeuten, während $R_2$ Methyl, Ethyl, Isopropyl oder sek.Butyl ist.

7. Verbindungen gemäss Anspruch 6, worin die Silylgruppe Trimethylsilyl, tris(tert.Butyl)silyl, Diphenyl-tert.-butylsilyl, bis(Isopropyl)-methylsilyl bedeutet.

8. Verbindungen gemäss Anspruch 6, worin die Silyl-Gruppe tert.Butyl-dimethylsilyl bedeutet.

9. Die Verbindung gemäss Anspruch 1 der Formel 2*, worin $R_1$ Wasserstoff ist und $R_2$ Ethyl bedeutet.

10. Die Verbindung gemäss Anspruch 1 der Formel 2*, worin $R_1$ Wasserstoff ist und $R_2$ Isopropyl bedeutet.

11. Verfahren zur Herstellung der Verbindungen der Formel 1* und 2* gemäss Anspruch 1, gekennzeichnet durch Reaktion von 14,15-Epoxi-Milbemycin-Derivaten der Formel 3*

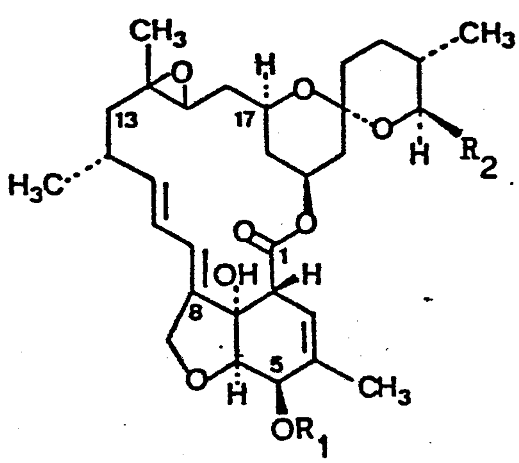

3*

worin $R_1$ und $R_2$ die für die Formeln $1^*$ und $2^*$ genannten Bedeutungen haben, in einer inerten trockenen Lösungsmittelphase bei -30°C bis +10°C mit einem Komplex-Reagenz $[HN_3]_m/Al(Ethyl_3]_n$, worin m und n unabhängig voneinander die Zahl 1 oder 2 oder einen Zahlenwert zwischen 1 und 2 darstellen.

12. Verfahren gemäss Anspruch 11, wobei im Temperaturbereich von -20°C bis -5°C gearbeitet wird.

13. Verfahren gemäss Anspruch 11, wobei unter Schutzgas gearbeitet wird.

14. Verfahren gemäss Anspruch 11, wobei die erhaltenen Endprodukte der Formeln $1^*$ und $2^*$ nach physikalisch-chemischen Methoden aufgetrennt werden.

15. Verfahren gemäss Anspruch 14, wobei die Endprodukte durch Chromatographie aufgetrennt werden.

16. Schädlingsbekämpfungsmittel enthaltend als Wirkstoff eine Verbindung der Formel 1* und/oder 2* gemäss Anspruch 1, zusammen mit einem geeigneten Trägerstoff.

17. Mittel gemäss Anspruch 16 enthaltend als Wirkstoff eine Verbindung gemäss einem der Ansprüche 2 bis 10.

18. Verwendung einer der Verbindungen der Formel $1^*$ oder $2^*$ des Anspruchs 1 zur Bekämpfung von Insekten und Schädlingen der Ordnung Acarina.

19. Verwendung einer der Verbindungen der Formel $1^*$ oder $2^*$des Anspruchs 1 zur Bekämpfung von Nematoden.

20. Verfahren zur Bekämpfung von Schädlingen an Tieren und Pflanzen, dadurch gekennzeichnet, dass man eine Verbindung der Formel $1^*$ oder $2^*$ gemäss Anspruch 1 auf oder in die Tiere bzw. Pflanzen appliziert.

21. Verfahren zur Bekämpfung von Ekto- und Endoparasiten am tierischen Warmblüter durch Applikation einer Verbindung der Formel 1* oder 2* gemäss Anspruch 1.

22. Epoxid-Derivate der Formel 3*

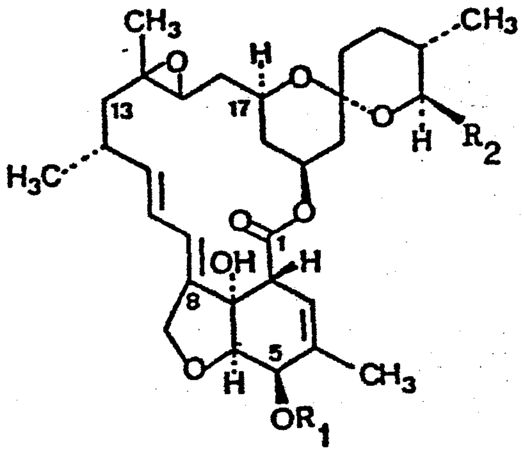

3*

worin $R_1$ eine Acyl- oder eine Silylgruppe und $R_2$ Methyl, Ethyl, Isopropyl oder sek.Butyl bedeuten, oder wobei $R_1$ Wasserstoff und $R_2$ sek.Butyl bedeuten.